# EUROPEAN PATENT SPECIFICATION

(11) **EP 4 225 772 B1**
(45) Date of publication and mention of the grant of the patent: **25.02.2026**
(21) Application number: 21801177.3
(22) Date of filing: 12.10.2021
(51) Int. Cl.: C07K 7/08, A61P 3/10, A61P 9/00, A61P 35/00, A61K 47/54, A61K 47/60, A61K 38/00

(54) **CYCLIC APELIN RECEPTOR AGONISTS**
ZYKLISCHE APELINREZEPTORAGONISTEN
AGONISTES DU RÉCEPTEUR DE L'APELINE CYCLIQUE

(30) Priority: 12.10.2020 GB 202016149
(43) Date of publication of application: 16.08.2023
(73) Proprietor: Nxera Pharma UK Limited, Cambridge, Cambridgeshire CB21 6DG (GB)
(72) Inventor: BROWN, Giles Albert, Cambridge Cambridgeshire CB21 6DG (GB); CONGREVE, Miles Stuart, Cambridge Cambridgeshire CB21 6DG (GB); SCULLY, Conor, Cambridge Cambridgeshire CB21 6DG (GB); PAUL, Rebecca, Cambridge Cambridgeshire CB21 6DG (GB)
(74) Representative: Gill Jennings & Every LLP
(86) International application number: PCT/GB2021/052634
(87) International publication number: WO 2022/079426

(56) References cited:
- WO-A1-2015/147641
- WO-A1-2019/005623
- WO-A2-2015/191781
- CN-A- 109 771 634
- GB-A- 2 551 945
- ANONYMOUS TAUTOMER: "Tautomer - Wikipedia", 20 December 2024 (2024-12-20), XP093235992, Retrieved from the Internet <URL:https://en.wikipedia.org/wiki/Tautomer> [retrieved on 20241220]
- WANG WANG ET AL: "Apelin protects against abdominal aortic aneurysm and the therapeutic role of neutral endopeptidase resistant apelin analogs", PROCEEDINGS OF THE NATIONAL ACADEMY OF SCIENCES, vol. 116, no. 26, 12 June 2019 (2019-06-12), pages 13006 - 13015, XP055876962, ISSN: 0027-8424, DOI: 10.1073/pnas.1900152116

## Description

This invention relates to a class of novel peptide compounds, their salts, and pharmaceutical compositions containing them. Said compounds, salts and compositions may be for use in therapy of the human body. In particular, the invention is directed to a class of compounds which are agonists of Apelin receptors. The disclosure also relates to the manufacture and use of these compounds and compositions in the prevention or treatment of such diseases in which Apelin receptors are involved.

The compounds relates to metabolically stable apelin analogs, covering and range of G protein-dependent and independent pharmacological profiles, and their use under both acute and chronic administration protocols, for the prevention or the treatment of disease mediated by the apelin receptor, in particular of cardiovascular disease (heart failure, kidney failure, hypertension, pulmonary hypertension, acute and chronic kidney injury and thrombotic diseases), diabetes, liver and gastrointestinal disease.

### Background of the Invention

Apelin is the endogenous ligand of the apelin receptor (also known as APJ, APLNR or angiotensin receptor-like 1). The Apelin receptor is a class A GPCR located on chromosome 11 consisting of 377 amino acids. To date only one apelin receptor has been identified in mammals, although two subtypes are present in amphibians and fish, and there are no closely related (homologous) genes.

In humans the APLN gene resides on chromosome X and encodes a 77 amino acid precursor preproapelin which is subsequently proteolytically cleaved to generate several isoforms: apelin-36, apelin-17, apelin-13 and [Pyr1] apelin-13. Among the isoforms [Pyr1] apelin-13 is the predominant isoform detected in human heart and plasma, however the plasma half life of apelin is very short (<5minutes) and therefore it is feasible additional short-lived isoforms with alternative structures and/or pharmacological properties may exist and potentially contribute to the physiological effects associated with the parent peptide apelin-36. Binding of the apelins to the apelin receptor can result in activation of multiple intracellular signaling pathways mediated by Gαi/o, Gα13 and possibly Gaq G proteins leading to recruitment of several signal transduction cascades including, but not limited to, phospholipase C (PLC), protein kinase C (PKC), AMP-activated protein kinase (AMPK), endothelial nitric oxide synthase, regulation of ERK1/2 phosphorylation and PI3K/Akt/p70S6 kinase signaling.

A second peptide of 54 amino acids Elabela/Toddler (ELABELA, or ELA, also known as Toddler, or Apela) has been identified which also activates the apelin receptor. The primary amino acid sequence of ELA does not demonstrate similarity to APJ however like APJ, ELA also undergoes rapid proteolytical cleavage to generate shorter isoforms. Both ligands are critical regulators of cardiovascular development and function.

Activation of the apelin receptor by endogenous ligands has also been demonstrated to result in the of β-arrestin, a protein that initiates receptor internalisation, desensitisation as well as downstream signalling. Recruitment of β-arrestin results in apparent short duration responses and an apelin receptor population that are refractory to further ligand-mediated activation. In various embodiments the identified examples can binding to and/or activate G proteinsignalling either alone or in combination with recruitment of β-arrestin thereby providing unique pharmacological profiles useful in the treatment of diseases related to apelin dysfunction.

Both apelin and APJ are relatively widely expressed across the central nervous system (CNS), peripheral tissues and blood, suggesting roles in multiple complex physiological processes. Based on multiple literature publications the apelin system has been implicated in roles in CNS disorders, thermoregulation, glucose homeostasis, angiogenesis, diabetes, pancreatitis, cardiovascular function, hepatic function and renal function, cancer (including but not limited to glioblastoma and colon cancer),

The APJ receptor and its ligands (apelin and ELA) have been implicated in the pathophysiology of human heart failure. Apelin receptors are present on endothelial cells, vascular smooth muscle cells and cardiomyocytes. Initial studies identified apelin as one of the most potent inotropic agents identified to date through direct actions on cardiomyocyte contractility without evidence of cardiac hypertrophy. Apelin has also been demonstrated to increase left ventricular contractility.

Apelin expression has been demonstrated to be altered in the setting of cardiovascular disease. An increase in apelin immunoreactivity has been observed in the plasma of patients in the early stages of heart failure, whereas a decrease is observed at later, more severe stages. Moreover, apelin receptor mRNA has been shown to be decreased in rat hypertrophied and failing hearts. Apelin gene-deficient mice were shown to develop an impaired heart contractility and progressive heart failure associated with aging and pressure overload. Therefore, down-regulation of the apelin system seems to coincide with declining cardiac performance raising the possibility that apelin could be a protective agent for cardiac function.

Systemic injection of apelin in rodents and humans has been demonstrated to result in significant decreases in blood pressure (BP) in rats via nitric oxide production. These data demonstrate that apelin exerts a hypotensive effect in vivo. However these effects on both blood pressure and inotropic cardiac output are short-lived, lasting only a few minutes, and demonstrating a degree of desensitization (also known as tachyphalaxis) leaving the apelin receptor refractory to further stimulation.

In chronic models of right ventricular failure apelin had inotropic effects and long-term treatment led to improved right ventricular mass, increased contractile force with decreased cardiac loading and hemodynamic measurements. Consistent with these findings apelin infusion has been demonstrated to improve pulmonary vascular hemodynamics in multiple preclinical models of pulmonary arterial hypertension (PAH) and these benefits have been confirmed to translate into PAH patients.

In zebrafish, ELA signalling is required for normal heart and vasculature development and its deficiency lead to severe defects in heart development and lymphogenesis. In humans ELA is expressed in adult embryonic stem cells and kidney and activates the human apelin receptor in respect of its activities to suppress cAMP production and to induce ERK1/2 phosphorylation and calcium mobilization. Functionally Elabela stimulates angiogenesis in human HUVECs and relaxes mouse aortic vessels.

In addition to a cardiovascular action of apelin, apelin receptor mRNA has been detected in all renal zones, most abundantly in the inner stripe of the outer medulla, in the glomeruli and a moderate expression was observed in all nephron segments, especially in collecting ducts. In agreement with this localization, the intravenous (iv) injection of apelin in increasing doses, dose-dependently increases diuresis.

Apelin expression has also been confirmed in human endothelial tissue where a key role in controlling fatty acid transport across the endothelial layer through apelin-induced inactivation of the transcription factor Forkhead box protein O1 (FOXO1) and subsequent inhibition of endothelial fatty acid binding protein 4 (FABP4) expression. These actions are consistent with predicted benefits on glucose utilisation and improved insulin sensitivity in diseases such as type 2 diabetes (T2DM).

Apelin receptor agonists may be useful alone and/or in combination with current standard of care treatments in the treatment of pulmonary arterial hypertension (PAH) increasing cardiac output, reducing pulmonary vessel hypertension, reducing inflammation, improve pulmonary tissue remodelling and preserving right heart ventricular function. PAH is a rare, progressive disorder characterized by high blood pressure (hypertension) in the arteries of the lungs (pulmonary artery) for no apparent reason. Symptoms of PAH include shortness of breath (dyspnea) especially during exercise, chest pain, and fainting episodes. The exact cause of PAH is unknown and although treatable, there is no known cure for the disease. PAH occurs twice as frequently in females as in males. It tends to affect females between the ages of 30 and 60. New cases are estimated to occur in one to two individuals per million each year in the U.S. The incidence is estimated to be similar in Europe. Approximately 500-1000 new cases of PAH are diagnosed each year in the U.S. There is no ethnic or racial group that is known to have a higher frequency of patients with PAH. Individuals with PAH may go years without a diagnosis, either because their symptoms are mild, nonspecific, or only present during demanding exercise. However, it is important to treat PAH because without treatment high blood pressure in the lungs causes the right heart to work much harder, and over time, this heart muscle may weaken or fail. The progressive nature of this disease means that an individual may experience only mild symptoms at first,but will eventually require treatment and medical care to maintain a normal lifestyle.

Apelin receptor agonists are agents useful in the treatment of cardiovascular conditions such as heart failure, acute decompensated heart failure, congestive heart failure, cardiomyopathy, ischemia, ischemia/reperfusion injury, fluid homeostasis, kidney failure, hypertension, pulmonary hypertension, polycystic kidney disease, hyponatremia and SIADH to increase cardiac output, improve cardiac function, stabilise cardiac function, limit further decrease in cardiac function, reduce systemic and portal hypertension, promote angiogenesis and new blood vessel formation in ischemic tissue, treat abnormalities in thrombosis and platelet function and improve kidney function and diuresis. Heart failure constitutes a major and growing health burden. In Europe there are at least 15 million patients with heart failure and in the United States, heart failure affects nearly 5,800,000 people. Heart failure incidence approaches 10 per 1,000 population after age 65. In the United States, heart failure causes 280,000 deaths annually, and the estimated direct and indirect cost of heart failure for 2010 is $39,2 billion. Treatment options depend on the type, cause, symptoms and severity of the heart failure, including treating the underlying causes and lifestyle changes. A number of medications are prescribed for heart failure, and most patients will take more than one drug. Apelin receptor agonists are likely to be used on top of existing agents Despite the advancements obtained in medical therapy, the death rate of heart failure remains high: almost 50% of people diagnosed with heart failure will die within 5 years.

Abnormalities in platelet function are associated with a range of thrombotic diseases such as peripheral arterial disease (PAD), acute coronary syndrome (ACS), myocardial infarction (MI), heart attacks (HA), stroke and atherosclerosis. Apelin and APJNR are expressed in human and mouse platelets and apelin knockout mice displayed a prothrombotic phenotype with increased platelet aggregation. Stimulation of platelets with apelin has been demonstrated to engage signalling pathways associated with calcium, nitric oxide and thromboxane production consistent with predicted benefits in these conditions.

Apelin receptor agonists are also agents useful for the treatment and management of diabetes and associated related metabolic conditions, diabetic complications (for example diabetic nephropathy, retinopathy, neuropathy, non-alcoholic fatty liver disease, non-alcoholic steatosis, portal hypertension) and conditions where stimulation and/or growth and/or endurance of muscle mass may be considered beneficial. Apelin has been demonstrated to be expressed in endothelial cells and improved glucose tolerance, enhances glucose utilisation by muscle, increases muscle insulin sensitivity and improves angiogenesis in tissue with poor local blood supply. Apelin-neuroprotection, where administration of apelin peptides promote neuronal survival and/or increased numbers of neurons, will be useful in conditions with neuronal loss of function, such as diabetic neuropathy.

The half-life of apelin in the blood circulation is around one minute, this invention aims at designing, synthesising and testing novel potent and stable drugs that activate the apelin/apelin receptor pathway. Embodiments contained herein exemplify the potential to specifically activate intracellular signaling pathways in a manner independent of β-arrestin activation and consistent with sustained receptor activation in the absence of desensitsation and/or tachyphalaxis. Such a compound constitutes a potential new therapeutic agent to treat diseases mediated by the apelin receptor as described in this invention.

WO 2015/191781 describes modified apelin polypeptides having increased stability, circulating half-life, and/or potency relative to the native apelin-13 polypeptide, as well as methods of using the polypeptides for treating cardiac disorders.

### Summary of the Invention

The present invention relates to novel compounds with agonist activity at the Apelin receptor, and pharmaceutical compositions comprising these. The compounds may be used for the manufacture of medicaments for treatment of diseases.

Accordingly, in one embodiment the invention provides a compound of the formula (1): 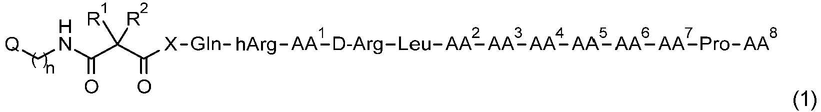 wherein;
Q is selected from phenyl or a monocyclic heteroaryl ring each of which may be optionally substituted with one or more R^{q} groups; or Q is a polyether chain of formula - (OCH₂CH₂)ₘOCH₃, wherein m is 1 to 5;
R^{q} is selected from halogen, hydroxyl, amino or C₁₋₆ alkyl having an alkyl chain optionally containing one or more heteroatoms selected from O, N, or S;
n is 1 to 3;
R¹ and R² are independently selected from hydrogen or a C₁₋₆ alkyl group, or together with the carbon to which they are attached join to form a C₃₋₈ cycloalkyl or a heterocyclyl group;
X is -DArg- or a bond;
-hArg- is a homoarginine residue;
AA¹ is the residue: 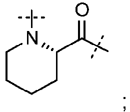
or is an aspartic acid derived residue joined to AA³ via a lactam bridge;
AA² is -Gly- or is a glutamic acid derived residue joined to AA⁵ via a lactam bridge;
AA³ is -His-, a 4-bromophenylalanine residue or is a lysine derived residue joined to AA¹ via a lactam bridge;
AA⁴ is the residue: 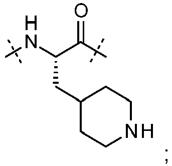
AA⁵ is -Gly- or is a lysine derived residue joined to AA² via a lactam bridge;
AA⁶ is the residue: 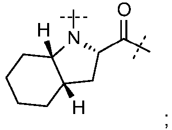
AA⁷ is a norleucine residue or a 4-bromophenylalanine residue;
AA⁸ is the residue: 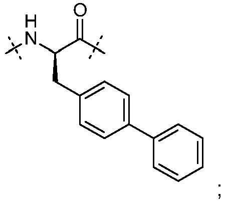
wherein the AA⁸ C-terminus is a carboxyl group or a carboxamide group and wherein the compound contains a lactam bridge;
or a tautomeric or stereochemically isomeric form thereof or a salt or zwitterion thereof.

### Detailed Description of the Invention

This invention relates to novel compounds. The compounds of the invention may be used as agonists of Apelin receptors and in the manufacture of medicaments for use as Apelin receptor agonists or for the treatment of disorders associated with Apelin receptors.

The compounds, compositions and medicaments of the invention may be useful for the treatment of disorders associated with Apelin receptors. Such disorders include cardiovascular disease, acute decompensated heart failure, congestive heart failure, myocardial infarction, cardiomyopathy, ischemia, ischemia/reperfusion injury, pulmonary hypertension, diabetes, obesity, cancer, metastatic disease, fluid homeostasis, pathological angiogenesis, retinopathy, HIV infection, treatment of pulmonary arterial hypertension (PAH) increasing cardiac output, reducing pulmonary vessel hypertension, reducing inflammation, improve pulmonary tissue remodelling, preserving right heart ventricular function, heart failure, congestive heart failure, cardiomyopathy, ischemia, ischemia/reperfusion injury, fluid homeostasis, kidney failure, hypertension, pulmonary hypertension, polycystic kidney disease, hyponatremia, SIADH, platelet function are associated with a range of thrombotic diseases such as peripheral arterial disease (PAD), acute coronary syndrome (ACS), myocardial infarction (MI), heart attacks (HA), stroke, atherosclerosis, treatment and management of diabetes and associated related metabolic conditions, diabetic complications (for example diabetic nephropathy, retinopathy, neuropathy, non-alcoholic fatty liver disease, non-alcoholic steatosis, portal hypertension) and conditions where stimulation and/or growth and/or endurance of muscle mass may be considered beneficial.

Also described herein is a method of treating the symptoms of various forms of central nervous system disorders including, dementia, including senile dementia and cerebrovascular dementia, depression, hyperkinetic (minimal brain damage) syndrome, disturbance of consciousness, anxiety disorder, schizophrenia, phobia, epilepsy, amyotrophic lateral sclerosis; Impairments of growth hormone secretion and/or function including but not limited to hyperphagia, polyphagia, hypercholesterolemia, hyperglyceridemia, hyperlipidemia, hyperprolactinemia, hypoglycemia, hypopituitarism, pituitary dwarfism; cancers, pancreatitis, renal diseases, Turner's syndrome, rheumatoid arthritis, spinal injury, spinocerebellar deformation, bone fractures, wounds, atopic dermatitis, osteoporosis, asthma, infertility, arteriosclerosis, pulmonary emphysema, pulmonary edema, and milk secretion insufficiency, and can also be used as a hypnotic sedative, a postoperative nutritional status improving agent, a preventive or therapeutic drug for HIV infection, AIDS, etc., and the like, comprising administering a Apelin acting polypeptide to a patient in need thereof.

Diseases or conditions for which the compounds may be beneficial include those selected from the group consisting of, treatment of pulmonary arterial hypertension (PAH) increasing cardiac output, reducing pulmonary vessel hypertension, reducing inflammation, improve pulmonary tissue remodelling and preserving right heart ventricular function, heart failure, congestive heart failure, cardiomyopathy, ischemia, ischemia/reperfusion injury, fluid homeostasis, kidney failure, hypertension, pulmonary hypertension, polycystic kidney disease, hyponatremia and SIADH, treatment and management of diabetes and associated related metabolic conditions, diabetic complications (for example diabetic nephropathy, retinopathy, neuropathy, non-alcoholic fatty liver disease, non-alcoholic steatosis, portal hypertension) and conditions where stimulation and/or growth and/or endurance of muscle mass.

A compound as outlined above may be used for the manufacture of a medicament for the treatment of any of the indications listed above.

Accordingly, in one embodiment the invention provides a compound of the formula (1): 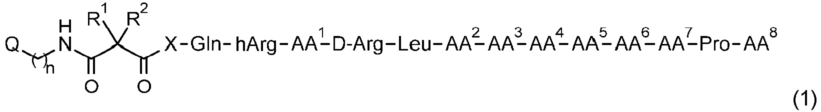 wherein;
Q is selected from phenyl or a monocyclic heteroaryl ring each of which may be optionally substituted with one or more R^{q} groups; or Q is a polyether chain of formula - (OCH₂CH₂)ₘOCH₃, wherein m is 1 to 5;
R^{q} is selected from halogen, hydroxyl, amino or C₁₋₆ alkyl having an alkyl chain optionally containing one or more heteroatoms selected from O, N, or S;
n is 1 to 3;
R¹ and R² are independently selected from hydrogen or a C₁₋₆ alkyl group, or together with the carbon to which they are attached join to form a C₃₋₈ cycloalkyl or a heterocyclyl group;
X is -DArg- or a bond;
-hArg- is a homoarginine residue;
AA¹ is the residue: 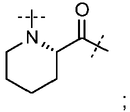
or is an aspartic acid derived residue joined to AA³ via a lactam bridge;
AA² is -Gly- or is a glutamic acid derived residue joined to AA⁵ via a lactam bridge;
AA³ is -His-, a 4-bromophenylalanine residue or is a lysine derived residue joined to AA¹ via a lactam bridge;
AA⁴ is the residue: 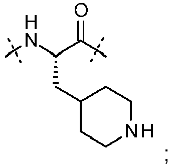
AA⁵ is -Gly- or is a lysine derived residue joined to AA² via a lactam bridge;
AA⁶ is the residue: 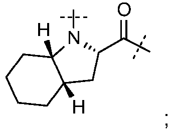
AA⁷ is a norleucine residue or a 4-bromophenylalanine residue;
AA⁸ is the residue: 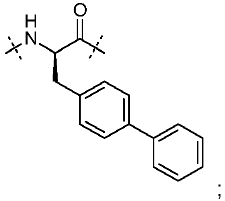
wherein the AA⁸ C-terminus is a carboxyl group or a carboxamide group and wherein the compound contains a lactam bridge;
or a tautomeric or stereochemically isomeric form thereof or a salt or zwitterion thereof.
Q can be selected from: 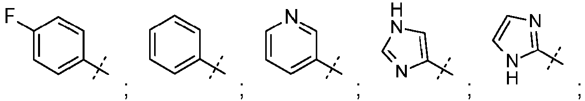 or 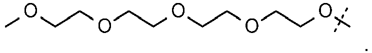
Q can be an imidazole ring. Q can be: 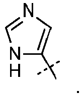
n can be 1. n can be 2. n can be 3.
R¹ and R² may be independently selected from hydrogen or a C₁₋₆ alkyl group. R¹ can be hydrogen or a C₁₋₆ alkyl group. R² can be hydrogen or a C₁₋₆ alkyl group. R¹ and R² can both be methyl. R¹ can be methyl. R² can be methyl.
X can be -DArg-. X can be a bond.
AA¹ can be the residue 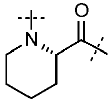 (homoproline). AA¹ can be an aspartic acid derived residue joined to AA³ via a lactam bridge. Where the lactam bridge is between AA¹ and AA³, AA² is -Gly- and AA⁵ is -Gly-.
AA² can be -Gly-. AA² can be a glutamic acid derived residue joined to AA⁵ via a lactam bridge. Where the lactam bridge is between AA² and AA⁵, AA¹ is -homoproline- and AA³ is - His- or 4-bromophenylalanine.
AA³ can be -His-. AA³ can be a 4-bromophenylalanine residue. AA³ can be a lysine derived residue joined to AA¹ via a lactam bridge. Where the lactam bridge is between AA¹ and AA³, AA² is -Gly- and AA⁵ is -Gly-.
AA⁵ can be -Gly-. AA⁵ can be a lysine derived residue joined to AA² via a lactam bridge. Where the lactam bridge is between AA² and AA⁵, AA¹ is -homoproline- and AA³ is -His- or 4-bromophenylalanine.
AA⁷ can be norleucine. AA⁷ can be 4-bromophenylalanine.

The AA⁸ C-terminus can be a carboxyl group. The AA⁸ C-terminus can be a carboxamide group.

All compounds described herein contain a single lactam bridge to internally cyclise the peptide sequence. The lactam bridge is between the side chain amino group of a lysine moiety at positions AA³ or AA⁵ and a side chain aspartic acid or glutamic acid at positions AA¹ or AA². Specifically the lactam bridge can be between an aspartic acid at AA¹ and a lysine at AA³. Alternatively the lactam bridge can be between a glutamic acid at AA² and a lysine at AA⁵. Where the lactam bridge is between AA¹ and AA³, AA² is -Gly- and AA⁵ is - Gly-. Where the lactam bridge is between AA² and AA⁵, AA¹ is -homoproline- and AA³ is - His- or 4-bromophenylalanine.

Particular examples of moiety 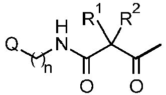 include caps 1-7 as shown below where the COOH group is coupled to the amine of the peptide X or AA¹ where X is a bond:

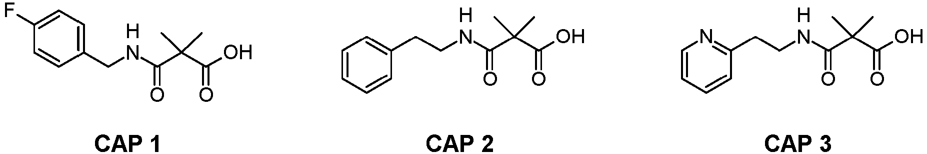

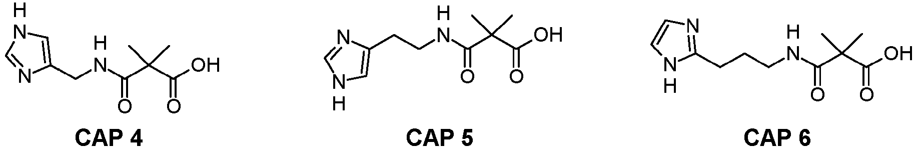

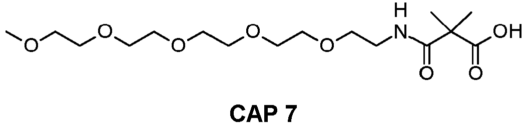

The compound can be selected from any one of Examples 1 to 6 as shown in Table 1.

Specific examples of compounds include compounds having Apelin receptor agonist activity.

The compounds of the invention may be used in a pharmaceutical composition comprising a compound of the invention and a pharmaceutically acceptable excipient.

The compounds of the invention may be used in medicine.

The compounds of the invention may be used in the treatment of disorders associated with Apelin receptors listed above.

### Definitions

In this application, the following definitions apply, unless indicated otherwise.

The term "alkyl", "aryl", "halogen", "cycloalkyl", "heterocyclyl" and "heteroaryl" are used in their conventional sense (e.g. as defined in the IUPAC Gold Book) unless indicated otherwise.

The term "treatment", in relation to the uses of any of the compounds described herein, including those of the formula (1), is used to describe any form of intervention where a compound is administered to a subject suffering from, or at risk of suffering from, or potentially at risk of suffering from the disease or disorder in question. Thus, the term "treatment" covers both preventative (prophylactic) treatment and treatment where measurable or detectable symptoms of the disease or disorder are being displayed.

The term "effective therapeutic amount" as used herein (for example in relation to methods of treatment of a disorder, disease or condition) refers to an amount of the compound which is effective to produce a desired therapeutic effect. For example, if the condition is pain, then the effective therapeutic amount is an amount sufficient to provide a desired level of pain relief. The desired level of pain relief may be, for example, complete removal of the pain or a reduction in the severity of the pain.

To the extent that any of the compounds described have chiral centres, the present invention extends to all optical isomers of such compounds, whether in the form of racemates or resolved enantiomers. The invention described herein relates to all crystal forms, solvates and hydrates of any of the disclosed compounds however so prepared. To the extent that any of the compounds disclosed herein have acid or basic centres such as carboxylates or amino groups, then all salt forms of said compounds are included herein. In the case of pharmaceutical uses, the salt should be seen as being a pharmaceutically acceptable salt.

Salts or pharmaceutically acceptable salts that may be mentioned include acid addition salts and base addition salts. Such salts may be formed by conventional means, for example by reaction of a free acid or a free base form of a compound with one or more equivalents of an appropriate acid or base, optionally in a solvent, or in a medium in which the salt is insoluble, followed by removal of said solvent, or said medium, using standard techniques (e.g. in vacuo, by freeze-drying or by filtration). Salts may also be prepared by exchanging a counter-ion of a compound in the form of a salt with another counter-ion, for example using a suitable ion exchange resin.

Examples of pharmaceutically acceptable salts include acid addition salts derived from mineral acids and organic acids, and salts derived from metals such as sodium, magnesium, potassium and calcium.

Examples of acid addition salts include acid addition salts formed with acetic, 2,2-dichloroacetic, adipic, alginic, aryl sulfonic acids (e.g. benzenesulfonic, naphthalene-2-sulfonic, naphthalene-1,5-disulfonic and p-toluenesulfonic), ascorbic (e.g. L-ascorbic), L-aspartic, benzoic, 4-acetamidobenzoic, butanoic, (+) camphoric, camphor-sulfonic, (+)-(1S)-camphor-10-sulfonic, capric, caproic, caprylic, cinnamic, citric, cyclamic, dodecylsulfuric, ethane-1,2-disulfonic, ethanesulfonic, 2-hydroxyethanesulfonic, formic, fumaric, galactaric, gentisic, glucoheptonic, gluconic (e.g. D-gluconic), glucuronic (e.g. D-glucuronic), glutamic (e.g. L-glutamic), α-oxoglutaric, glycolic, hippuric, hydrobromic, hydrochloric, hydriodic, isethionic, lactic (e.g. (+)-L-lactic and (±)-DL-lactic), lactobionic, maleic, malic (e.g. (-)-L-malic), malonic, (±)-DL-mandelic, metaphosphoric, methanesulfonic, 1-hydroxy-2-naphthoic, nicotinic, nitric, oleic, orotic, oxalic, palmitic, pamoic, phosphoric, propionic, L-pyroglutamic, salicylic, 4-amino-salicylic, sebacic, stearic, succinic, sulfuric, tannic, tartaric (e.g.(+)-L-tartaric), thiocyanic, undecylenic and valeric acids.

Also encompassed are any solvates of the compounds and their salts. Preferred solvates are solvates formed by the incorporation into the solid state structure (e.g. crystal structure) of the compounds of the invention of molecules of a non-toxic pharmaceutically acceptable solvent (referred to below as the solvating solvent). Examples of such solvents include water, alcohols (such as ethanol, isopropanol and butanol) and dimethylsulfoxide. Solvates can be prepared by recrystallising the compounds of the invention with a solvent or mixture of solvents containing the solvating solvent. Whether or not a solvate has been formed in any given instance can be determined by subjecting crystals of the compound to analysis using well known and standard techniques such as thermogravimetric analysis (TGA), differential scanning calorimetry (DSC) and X-ray crystallography.

The solvates can be stoichiometric or non-stoichiometric solvates. Particular solvates may be hydrates, and examples of hydrates include hemihydrates, monohydrates and dihydrates. For a more detailed discussion of solvates and the methods used to make and characterise them, see Bryn et al, Solid-State Chemistry of Drugs, Second Edition, published by SSCI, Inc of West Lafayette, IN, USA, 1999, ISBN 0-967-06710-3.

The term "pharmaceutical composition" in the context of this invention means a composition comprising an active agent and comprising additionally one or more pharmaceutically acceptable carriers. The composition may further contain ingredients selected from, for example, diluents, adjuvants, excipients, vehicles, preserving agents, fillers, disintegrating agents, wetting agents, emulsifying agents, suspending agents, sweetening agents, flavouring agents, perfuming agents, antibacterial agents, antifungal agents, lubricating agents and dispersing agents, depending on the nature of the mode of administration and dosage forms. The compositions may take the form, for example, of tablets, dragees, powders, elixirs, syrups, liquid preparations including suspensions, sprays, inhalants, tablets, lozenges, emulsions, solutions, cachets, granules, capsules and suppositories, as well as liquid preparations for injections, including liposome preparations.

The compounds of the invention may contain one or more isotopic substitutions, and a reference to a particular element includes within its scope all isotopes of the element. For example, a reference to hydrogen includes within its scope ¹H, ²H (D), and ³H (T). Similarly, references to carbon and oxygen include within their scope respectively ¹²C, ¹³C and ¹⁴C and ¹⁶O and ¹⁸O. In an analogous manner, a reference to a particular functional group also includes within its scope isotopic variations, unless the context indicates otherwise. For example, a reference to an alkyl group such as an ethyl group or an alkoxy group such as a methoxy group also covers variations in which one or more of the hydrogen atoms in the group is in the form of a deuterium or tritium isotope, e.g. as in an ethyl group in which all five hydrogen atoms are in the deuterium isotopic form (a perdeuteroethyl group) or a methoxy group in which all three hydrogen atoms are in the deuterium isotopic form (a trideuteromethoxy group). The isotopes may be radioactive or non-radioactive.

Therapeutic dosages may be varied depending upon the requirements of the patient, the severity of the condition being treated, and the compound being employed. Determination of the proper dosage for a particular situation is within the skill of the art. Generally, treatment is initiated with the smaller dosages which are less than the optimum dose of the compound. Thereafter the dosage is increased by small increments until the optimum effect under the circumstances is reached. For convenience, the total daily dosage may be divided and administered in portions during the day if desired.

The magnitude of an effective dose of a compound will, of course, vary with the nature of the severity of the condition to be treated and with the particular compound and its route of administration. The selection of appropriate dosages is within the ability of one of ordinary skill in this art, without undue burden. In general, the daily dose range may be from about 10 µg to about 30 mg per kg body weight of a human and non-human animal, preferably from about 50 µg to about 30 mg per kg of body weight of a human and non-human animal, for example from about 50 µg to about 10 mg per kg of body weight of a human and non-human animal, for example from about 100 µg to about 30 mg per kg of body weight of a human and non-human animal, for example from about 100 µg to about 10 mg per kg of body weight of a human and non-human animal and most preferably from about 100 µg to about 1 mg per kg of body weight of a human and non-human animal.

### Pharmaceutical Formulations

While it is possible for the active compound to be administered alone, it is preferable to present it as a pharmaceutical composition (e.g. formulation).

Accordingly, in another embodiment of the invention, there is provided a pharmaceutical composition comprising at least one compound of the formula (1) as defined above together with at least one pharmaceutically acceptable excipient.

The composition may be a composition suitable for injection. The injection may be intravenous (IV) or subcutaneous. The composition may be supplied in a sterile buffer solution or as a solid which can be suspended or dissolved in sterile buffer for injection.

The pharmaceutically acceptable excipient(s) can be selected from, for example, carriers (e.g. a solid, liquid or semi-solid carrier), adjuvants, diluents (e.g solid diluents such as fillers or bulking agents; and liquid diluents such as solvents and co-solvents), granulating agents, binders, flow aids, coating agents, release-controlling agents (e.g. release retarding or delaying polymers or waxes), binding agents, disintegrants, buffering agents, lubricants, preservatives, anti-fungal and antibacterial agents, antioxidants, buffering agents, tonicityadjusting agents, thickening agents, flavouring agents, sweeteners, pigments, plasticizers, taste masking agents, stabilisers or any other excipients conventionally used in pharmaceutical compositions.

The term "pharmaceutically acceptable" as used herein means compounds, materials, compositions, and/or dosage forms which are, within the scope of sound medical judgment, suitable for use in contact with the tissues of a subject (e.g. a human subject) without excessive toxicity, irritation, allergic response, or other problem or complication, commensurate with a reasonable benefit/risk ratio. Each excipient must also be "acceptable" in the sense of being compatible with the other ingredients of the formulation.

Pharmaceutical compositions containing compounds of the formula (1) can be formulated in accordance with known techniques, see for example, Remington's Pharmaceutical Sciences, Mack Publishing Company, Easton, PA, USA.

Suitable formulations typically contain 0-20% (w/w) buffers, 0-50% (w/w) cosolvents, and/or 0-99% (w/w) Water for Injection (WFI) (depending on dose and if freeze dried). Formulations for intramuscular depots may also contain 0-99% (w/w) oils.

The compounds of the formula (1) will generally be presented in unit dosage form and, as such, will typically contain sufficient compound to provide a desired level of biological activity. For example, a formulation may contain from 1 nanogram to 2 grams of active ingredient, e.g. from 1 nanogram to 2 milligrams of active ingredient. Within these ranges, particular subranges of compound are 0.1 milligrams to 2 grams of active ingredient (more usually from 10 milligrams to 1 gram, e.g. 50 milligrams to 500 milligrams), or 1 microgram to 20 milligrams (for example 1 microgram to 10 milligrams, e.g. 0.1 milligrams to 2 milligrams of active ingredient).

The active compound will be administered to a patient in need thereof (for example a human or animal patient) in an amount sufficient to achieve the desired therapeutic effect (effective amount). The precise amounts of compound administered may be determined by a supervising physician in accordance with standard procedures.

### EXAMPLES

The invention will now be illustrated, but not limited, by reference to the specific embodiments described in the following examples.

### EXAMPLES 1 TO 6

| | |
|---|---|
| 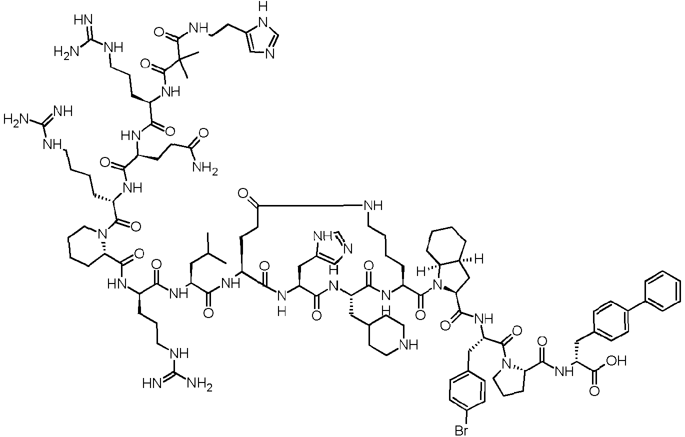 | 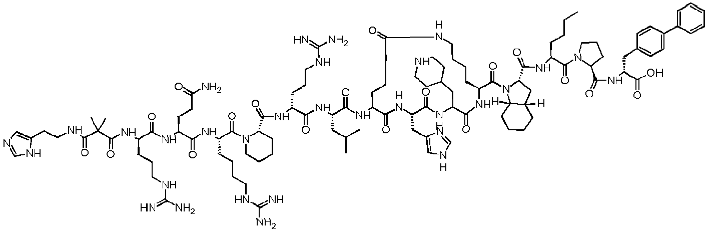 |
| **Example 1** | **Example 2** |
| 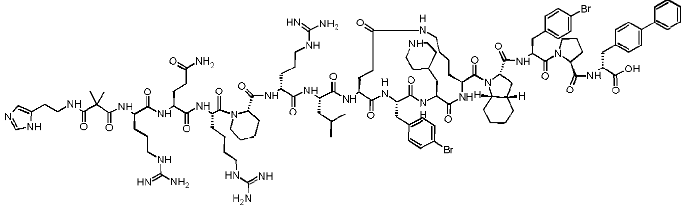 | 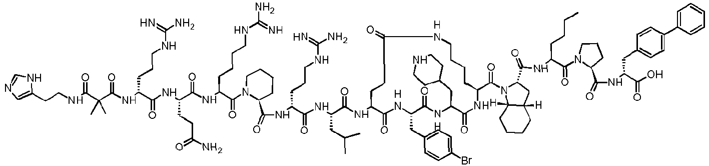 |
| **Example 3** | **Example 4** |
| 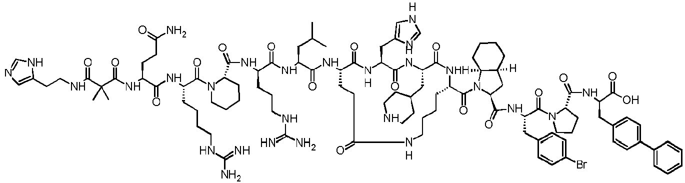 | 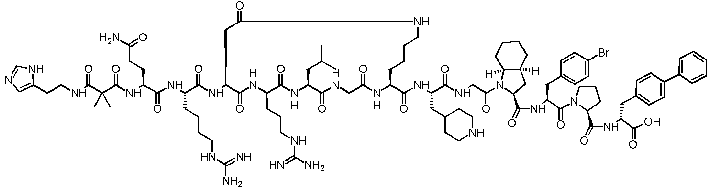 |
| **Example 5** | **Example 6** |

The compounds of Examples 1 to 6 shown in Table 1 below have been prepared. Their LCMS properties and the methods used to prepare them are set out in Table 2. The starting materials for each of the Examples are commercial unless indicated otherwise.

**Table 1**

| | **1** | **2** | **3** | **4** | **5** | **6** | **7** | **8** | **9** | **10** | **11** | **12** | **13** | **14** | **15** | **16** |
|---|---|---|---|---|---|---|---|---|---|---|---|---|---|---|---|---|
| Example 1 | CAP 5 | D-ARG | GLN | homoARG | homoPRO | D-ARG | LEU | CycloGLU | HIS | PipALA | CycloLYS | OIC | 4-BrPHE | PRO | D-BIP | OH |
| Example 2 | CAP 5 | D-ARG | GLN | HomoARG | HomoPRO | D-ARG | LEU | CycloGLU | HIS | PipALA | CycloLYS | OIC | NLE | PRO | D-BIP | OH |
| Example 3 | CAP 5 | D-ARG | GLN | HomoARG | HomoPRO | D-ARG | LEU | CycloGLU | 4-BrPHE | PipALA | CycloLYS | OIC | 4-BrPHE | PRO | D-BIP | OH |
| Example 4 | CAP 5 | D-ARG | GLN | HomoARG | HomoPRO | D-ARG | LEU | CycloGLU | 4-BrPHE | PipALA | CycloLYS | OIC | NLE | PRO | D-BIP | OH |
| Example 5 | | CAP 5 | GLN | HomoARG | HomoPRO | D-ARG | LEU | CycloGLU | HIS | PipALA | CycloLYS | OIC | 4-BrPHE | PRO | D-BIP | OH |
| Example 6 | | CAP 5 | GLN | HomoARG | CycloASP | D-ARG | LEU | GLY | CycloLYS | PipALA | GLY | OIC | 4-BrPHE | PRO | D-BIP | OH |

Standard amino acid symbols are used in Table 1 where appropriate. In cases where a standard symbol is not available, the following representations are used:

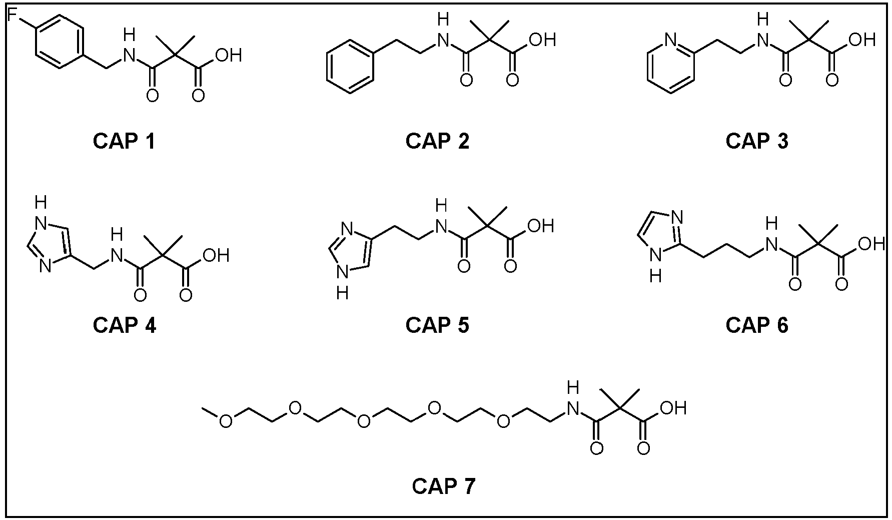

Standard amino acid symbols are used in Table 1 where appropriate. In cases where a standard symbol is not available, the following representations are used:

| | | |
|---|---|---|
| 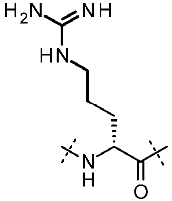 D-ARG | 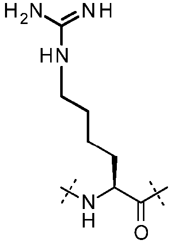 homoARG | 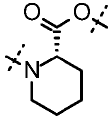 homoPRO |
| 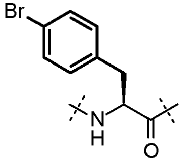 4-BrPHE | 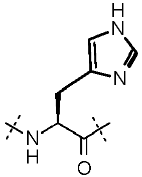 HIS | 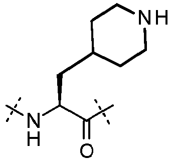 PipALA |
| 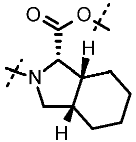 OIC | 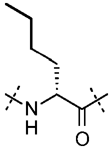 NorLEU/NLE | 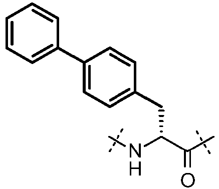 D-BIP |

### General procedures

Where no preparative routes are included, the relevant intermediate is commercially available. Commercial reagents were utilized without further purification. Room temperature (rt) refers to approximately 20-27°C. ¹H NMR spectra were recorded at 400 MHz on a Bruker instrument. Chemical shift values are expressed in parts per million (ppm), i.e. (δ)-values. The following abbreviations are used for the multiplicity of the NMR signals: s=singlet, br=broad, d=doublet, t=triplet, q=quartet, quint=quintet, td=triplet of doublets, tt= triplet of triplets, qd=quartet of doublets, ddd=doublet of doublet of doublets, ddt=doublet of doublet of triplets, m=multiplet. Coupling constants are listed as *J* values, measured in Hz. NMR and mass spectroscopy results were corrected to account for background peaks. Chromatography refers to column chromatography performed using 60 - 120 mesh silica gel and executed under nitrogen pressure (flash chromatography) conditions.

### Analytical Methods

LCMS analysis of compounds was performed under electrospray conditions

### LCMS Method A

Instruments: Waters Acquity UPLC, Waters 3100 PDA Detector, SQD; Column: Acquity HSS-T3, 1.8 micron, 2.1 x 100 mm; Gradient [time (min)/solvent B in A (%)]: 0.00/10, 1.00/10, 2.00/15, 4.50/55, 6.00/90, 8.00/90, 9.00/10, 10.00/10; Solvents: solvent A = 0.1% trifluoroacetic acid in water; solvent B = acetonitrile; Injection volume 1µL; Detection wavelength 214 nm; Column temperature 30 °C; Flow rate 0.3 mL per min.

### Analytical Method B

MS ion determined using LCMS method below under electrospray conditions, HPLC retention time (R_{T}) determined using HPLC method below, purity > 95% by HPLC unless indicated.

LCMS: Agilent 1200 HPLC&6410B Triple Quad, Column: Xbridge C18 3.5µm 2.1*30mm. Gradient [time (min)/solvent B(%)]:0.0/10,0.9/80,1.5/90,8.5/5,1.51/10. (Solvent A=1mL of TFA in 1000 mL Water; Solvent B=1mL of TFA in 1000 mL of MeCN); Injection volume 5 µL (may vary); UV detection 220 nm 254 nm 210 nm; Column temperature 25°C; 1.0 mL/min.

HPLC: Agilent Technologies 1200, Column: Sepax GP-C18 5µm 120A 4.6*150mm. Gradient [time (min)/solvent B(%)]:0.0/40,20/55,20.1/90,23/90. (Solvent A=1mL of TFA in 1000 mL Water; Solvent B=1mL of TFA in 1000 mL of 80%MeCN+20%H2O); Injection volume 30 µL (may vary); UV detection 220 nm; Column temperature 25°C; 1.0 mL/min

### Analytical Method C

MS ion determined using LCMS method below under electrospray conditions, HPLC retention time (R_{T}) determined using HPLC method below, purity > 95% by HPLC unless indicated.

LCMS: Agilent 1200 HPLC&6410B Triple Quad, Column: Xbridge C18 3.5um 2.1*30mm. Gradient [time (min)/solvent B(%)]:0.0/10,0.9/80,1.5/90,8.5/5,1.51/10. (Solvent A=1mL of TFA in 1000 mL Water; Solvent B=1mL of TFA in 1000 mL of MeCN); Injection volume 5 µL (may vary); UV detection 220 nm 254 nm 210 nm; Column temperature 25°C; 1.0 mL/min.

HPLC: Agilent Technologies 1200, Column: Gemini-NX C18 5um 110A 150*4.6mm. Gradient [time (min)/solvent B(%)]:0.0/30,20/60,20.1/90,23/90. (Solvent A=1mL of TFA in 1000 mL Water; Solvent B=1mL of TFA in 1000 mL of MeCN); Injection volume 5 µL (may vary); UV detection 220 nm 254 nm; Column temperature 25°C; 1.0 mL/min

### Analytical Method D

Instrument: Thermo Scientific Orbitrap Fusion; Column: Phenomenex Kinetex Biphenyl 100 Å, 2.6 µm, 2.1 x 50 mm; Gradient [time (min)/solvent B in A (%)]: 0.00/10, 0.30/10, 0.40/60, 1.10/90, 1.70/90, 1.75/10, 1.99/10, 2.00/10; Solvents: Solvent A = 0.1% formic acid in water; Solvent B = 0.1% formic acid in acetonitrile; Injection volume 5 µL; Column temperature 25 \°C; Flow rate 0.8 mL/min.

### Synthesis of Intermediates and Compounds

The following examples are provided to illustrate preferred aspects of the invention and are not intended to limit the scope of the invention.

### Synthesis of Intermediates

All Fmoc-amino acids are commercially available except for the Intermediate 1 building block, the synthesis of which is outlined below

### Synthesis of 2,2-dimethyl-3-oxo-3-((2-(1-trityl-1H-imidazol-4-yl)ethyl)amino)propanoic acid (Intermediate 1)

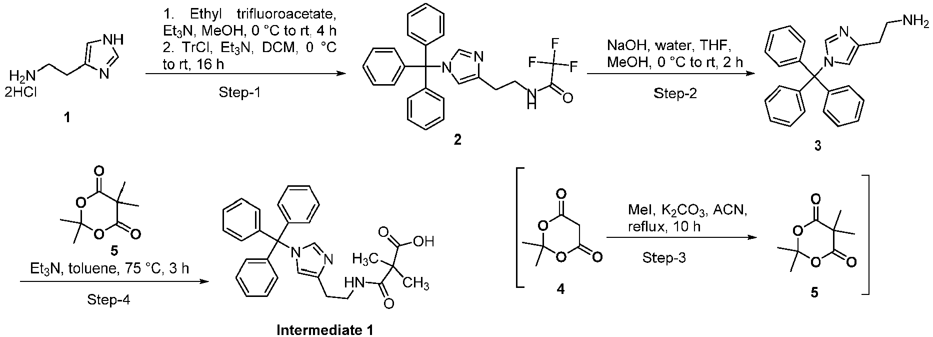

**Step-1: Synthesis of 2,2,2-trifluoro-*N*-(2-(1-trityl-1*H*-imidazol-4-yl)ethyl)acetamide (2):** To a solution of 2-(1H-imidazol-4-yl)ethan-1-amine dihydrochloride (1, 25.0 g, 136.6 mmol) in MeOH (100 mL), Et₃N (67 mL, 464.4 mmol) was added at rt and the reaction mixture was cooled to 0 °C. A solution of ethyl trifluoroacetate (20 mL, 164.0 mmol) in MeOH (50 mL) was added to the reaction mixture over 30 min at 0 °C and the reaction mixture was stirred at rt for 4 h. This reaction mixture was diluted with dry DCM (200 mL) and Et₃N (60 mL, 409.8 mmol) and the reaction mixture was cooled to 0 °C. Tr-Cl (76 g, 273.2 mmol) was added portion wise and the resulting reaction mixture was stirred at rt for 16 h. After completion, the reaction mixture was quenched with water (300 mL) and the aq layer was extracted with chloroform (3 x 150 mL). The organic layers were combined, dried (Na₂SO₄) and concentrated in *vacuo.* The crude residue was triturated with n-hexane to give 2,2,2-trifluoro-N-(2-(1-trityl-1H-imidazol-4-yl)ethyl)acetamide (2, 50.10 g, 81%) as a white solid.
MS (ESI +ve): 450
**¹H-NMR** (400 MHz; CDCl₃): δ 2.75 (t, *J* = 5.9 Hz, 2H), 3.60 - 3.65 (m, 2H), 6.61 (s, 1H), 7.08 - 7.15 (m, 6H), 7.31 - 7.38 (m, 9H), 7.40 (s, 1H), 8.41 (bs, 1H).

**Step-2: Synthesis of 2-(1-trityl-1*H*-imidazol-4-yl)ethan-1-amine (3):** To a solution of 2,2,2-trifluoro-N-(2-(1-trityl-1*H*-imidazol-4-yl)ethyl)acetamide (2, 50.0 g, 111.3 mmol) in THF (150 mL) and MeOH (180 mL), NaOH (22.0 g, 556.7 mmol) in water (100 mL) was slowly added at 0 °C and the reaction mixture was stirred at room temperature for 2 h. After completion, the reaction mixture was quenched with water (300 mL) and the aq layer was extracted with chloroform (3 x 150 mL). The organic layers were combined, dried (Na₂SO₄) and concentrated in *vacuo* to give 2-(1-trityl-1*H*-imidazol-4-yl)ethan-1-amine (3, 34.0 g, 86%) as a yellowish sticky solid. The crude residue was used for the next step without further purification.
**MS (ESI +ve):** 354
**¹H-NMR** (400 MHz; CDCl₃): δ1.53 (bs, 2H), 2.65 (t, *J* = 6.5 Hz, 2H), 2.95 (t, *J* = 6.5 Hz, 2H), 6.58 (s, 1H), 7.11 - 7.16 (m, 6H), 7.28 - 7.38 (m, 10H).

**Step-3: Synthesis of 2,2,5,5-tetramethyl-1,3-dioxane-4,6-dione** (5): To a solution of 2,2-dimethyl-1,3-dioxane-4,6-dione (**4**, 20.0 g, 138.8 mmol) in ACN (200 mL), K₂CO₃ (96 g, 694.0 mmol) and Mel (26 mL, 416.6 mmol) were added at rt and reaction mixture was refluxed for 10 h. After completion, the reaction mixture was cooled to room temperature, filterd through a pad of celite, washed with EtOAc (3 x 50 mL). The organic layer was washed with 10% aq Na₂S₂O₃ (100 mL), dried, (Na₂SO₄) and concentrated in *vacuo* to give 2,2,5,5-tetramethyl-1,3-dioxane-4,6-dione (5, 21 g, 88%) as a yellow solid. The crude residue was used for the next step without further purification.

**¹H-NMR** (400 MHz; CDCl₃): *δ* 1.63 (s, 6H), 1.73 (s, 6H).

**Step-4: Synthesis of 2,2-dimethyl-3-oxo-3-((2-(1-trityl-1*H*-imidazol-4-yl)ethyl)amino) propanoic acid (Intermediate 1):** A solution of 2-(1-trityl-1*H*-imidazol-4-yl)ethan-1-amineto (3, 8.0 g, 22.6 mmol) and Et₃N (16.0 mL, 113.0 mmol) in toluene (100 mL) was added drop wise over 60 min to a solution of 2,2,5,5-tetramethyl-1,3-dioxane-4,6-dione (5, 5.8 g, 29.76 mmol) in toluene (50 mL) at 75 °C. The reaction mixture was further stirred at same temperature was 3 h. After completion, the reaction mixture was concentrated in *vacuo.* The residue was dissolved in chloroform (100 mL) and washed with 10% aq citric acid (pH ~ 6 - 6.5). The organic layer was dried (Na₂SO₄) and concentrated in *vacuo.* The crude residue obtained was triturated with hot chloroform (150 mL) and n-hexane (75 mL) and the suspension was stirred at rt for 16 h. The solid was filtered, washed with chloroform : n-hexane (1:1, 2 x 50 mL) and dried in *vacuo* to give 2,2-dimethyl-3-oxo-3-((2-(1-trityl-1*H*-imidazol-4-yl)ethyl)amino)propanoic acid (**Intermediate 1,** 6.8 g, 64%) as a white solid.
**LCMS (Method A):** m/z 468 [M+H]⁺ (ES⁺), at 5.38 min, 99.31%
**¹H-NMR** (400 MHz; DMSO-d₆): *δ* 1.21 (s, 6H), 2.57 (t, *J* = 6.8 Hz, 2H), 3.22 - 3.27 (m, 2H), 6.66 (s, 1H), 7.06 - 7.11 (m, 6H), 7.28 (s, 1H), 7.35 - 7.42 (m, 8H), 7.64 (t, *J* = 5.4 Hz, 1H), 8.31 (s, 1H), 12.44 (bs, 1H).

### Synthesis of Examples 1-6

Standard Fmoc solid phase peptide synthesis (SPPS) was used to synthesize the linear peptides which were then cleaved from the resin and purified.

**General method for Peptide Synthesis:** The peptide was synthesized using standard Fmoc chemistry.

### Method a - Exemplified by the Synthesis of Example 2

### Peptide Synthesis

1) Add DCM to the vessel containing CTC Resin (0.2 mmol) and Fmoc-D-Bip-OH (92.4 mg, 0.2 mmol, 1.0 eq) agitate with N₂ bubbling.
2) Drain and then wash with DMF (5 times, drain between each wash).
3) A solution of 20% piperidine in DMF was added agitate with N₂ bubbling for 30 min.
4) Drain and wash with DMF (5 times, drain between each wash).
5) Add Fmoc-amino acid solution (3.0 equivalents in DMF) and mix for 30 seconds, then add activation buffer (HBTU (2.85 equivalents) and DIEA (6 equivalents) in DMF), agitate with N₂ bubbling for 1 hour.
6) The coupling reaction was monitored by ninhydrin test
7) If required repeat steps 4 to 6 for same amino acid coupling if inefficient coupling occurs
8) Repeat steps 2 to 6 for next amino acid coupling.
Note: for the acids in the table below different protecting groups and / or coupling agents were used

| **Step** | **Materials** | **Coupling reagents** |
|---|---|---|
| 5 | Fmoc-Lys(Alloc)-OH (3.0 eq) | HBTU (2.85 eq) and DIEA (6.0 eq) |
| 8 | Fmoc-Glu(OAII)-OH (3.0 eq) | HBTU (2.85 eq) and DIEA (6.0 eq) |
| 15 | Intermediate 1 (2.0 eq) | HOAt (2.0 eq) and DIC (2.0 eq) |

### Peptide sidechain deprotection cyclisation:

1) Add DCM to the resin and agitate with N₂ bubbling, then add PhSiH₃ (10 eq), Pd(PPh₃)₄ (0.2 eq) agitate with N₂ for 15 mins for 3 times.
2) The resin was washed with DCM three times and then DMF three times.
3) The resin was washed with 0.5% Sodium diethyldithiocarbamate trihydrate DMF and 0.5% DIEA in DMF for ten times.
4) HATU (2 eq) and DIEA (4 eq) were added to the resin in DMF and agitate with N₂ bubbling for 1 hour.
5) The resin was washed with MeOH three times and dried *in vacuo.*

### Peptide Cleavage and Purification:

1) Add cleavage buffer (92.5%TFA/2.5%EDT/2.5%TIS/2.5%H₂O) to the flask containing the side chain protected peptide on resin at room temperature and stir for 3 hours.
2) Filter and collect the peptide solution.
3) The peptide is precipitated with cold tert-butyl methyl ether and centrifuged (3 min at 3000 rpm).
4) Residue washed with tert-butyl methyl ether (2 times).
5) Crude peptide dried under vacuum for 2 hours.
6) The crude peptide was purified by prep-HPLC. Prep-HPLC Conditions: Instrument: Gilson 281. Solvent: A- 0.1% TFA in H2O, B- acetonitrile, Column: Luna C18 (200×25 mm; 10 µm) and Gemini C18 (150*30 mm; 5 µm) in series. Gradient [time (min)/solvent B (%)]:0.0/60, 60.0/90, 60.1/90, 70/90, 70.1/10, at 20 mL/min with UV detection (wave length = 215/254 nm) and then lyophilized to give Example 2 (28.8 mg, 6.62% yield).

**Table 2 - HRMS and LCMS properties of purify peptides represented by Examples 1-23**

| **Example** | **HRMS (Method D)** | **Analytical Method** | **LCMS / HPLC** |
|---|---|---|---|
| **1** | HRMS (HESI/FT) m/z: [M+4H]⁴⁺ Calcd for C109H160O19N31Br 2286.169; Found 572.5543 | B | m/z 1145.0 [M+2H]²⁺, R_{T} = 11.31 min |
| **2** | HRMS (HESI/FT) m/z: [M+4H]⁴⁺ Calcd for C106H163O19N31 2174.2742; Found 544.5818 | C | m/z 726.1 [M+3H]³⁺, R_{T} = 7.26 min |
| **3** | HRMS (HESI/FT) m/z: [M+4H]⁴⁺ Calcd for C112H161O19N29Br2374.0891; Found 594.5337 | C | m/z 793.2 [M+3H]³⁺, R_{T} = 11.57min |
| **4** | HRMS (HESI/FT) m/z: [M+4H]⁴⁺ Calcd for C109H164O19N29Br2262.1941; Found 566.5570 | C | m/z 755.7 [M+3H]³⁺, R_{T} = 9.89 min |
| **5** | HRMS (HESI/FT) m/z: [M+4H]⁴⁺ Calcd for C103H148O18N27Br 2130.0679; Found 533.5250 | C | m/z 711.8 [M+3H]³⁺, R_{T} = 9.83 min |
| **6** | HRMS (HESI/FT) m/z: [M+4H]⁴⁺ Calcd for C94H136O18N25Br 1981.9679; Found 496.4998 | C | m/z 662.2 [M+3H]³⁺, R_{T} = 7.43 min |

| | | | |
|---|---|---|---|
| ND - Not determined | | | |

### Biological Activity

The following examples are provided to illustrate preferred aspects of the invention and are not intended to limit the scope of the invention.

### Example A. In vitro pharmacological characterization of Apelin peptides - Functional agonism of human Apelin receptors, cAMP accumulation assay:

**cAMP functional assay.** cAMP production was quantified using the Homogeneous Time-Resolved Fluorescence (HTRF) cAMP dynamic-2 assay (Cisbio, France). CHO cells stably expressing the human Apelin receptor were seeded at a density of 12,500 cells/well in solid walled 96 well half area plates (Costar). After 16 h incubation at 37°C media was removed and cells were incubated at 37°C for 30 min in serum free media containing 500 µM IBMX (Tocris), 3uM forskolin to raise cAMP levels and increasing concentrations of test agonist. cAMP production was determined as manufacturer's instructions before plates were read on a PheraStar fluorescence plate reader (BMG LabTech) and EC₅₀ values were determined using Graphpad Prism.

| Example | Human Apelin agonist cAMP assay | |
|---|---|---|
| | **pEC₅₀** | **Emax** |
| Apelin | 8.9 | 112.8 |
| **1** | 8.5 | 70.5 |
| **2** | 7.8 | 31.2 |
| **3** | 8.1 | 27.9 |
| **4** | 7.0 | 28.4 |
| **5** | 8.3 | 48.2 |
| **6** | 7.9 | 30.5 |

### Example B. In vitro pharmacological characterization of Apelin peptides - Functional agonism of human Apelin receptors, β-arrestin accumulation assay:

**β-arrestin assay.** CHO-K1 cells engineered to overexpress the human Apelin receptor and β-arrestin (DiscoverRx) were seeded at a density of 12,500 cells/well in solid walled 96 well half area plates (Costar). After 16 h incubation at 37°C media was removed and cells were incubated at 37°C for 90 min in serum free media containing increasing concentrations of test agonist. The assay reaction was stopped by adding detection reagent (DiscoveRx) and incubation for 60 min in the dark. Levels of receptor activation were then measured on a PheraStar fluorescence plate reader (BMG LabTech) and EC₅₀ values were determined using Graphpad Prism. Emax value only reported for active compounds.

| Example | Human Apelin agonist β-arrestin assay |
|---|---|
| | **pEC₅₀ (Emax)** |
| Apelin | 8.6 (165.8) |
| **1** | <5 |
| **2** | <5 |
| **3** | <5 |
| **4** | <5 |
| **5** | <5 |
| **6** | <4 |

## Claims

1. A compound comprising the sequence of formula (1): 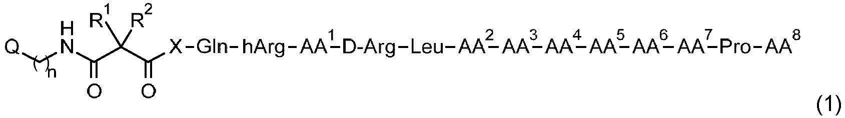 wherein;
Q is selected from phenyl or a monocyclic heteroaryl ring each of which may be optionally substituted with one or more R^{q} groups; or Q is a polyether chain of formula - (OCH₂CH₂)ₘOCH₃, wherein m is 1 to 5;
R^{q} is selected from halogen, hydroxyl, amino or C₁₋₆ alkyl having an alkyl chain optionally containing one or more heteroatoms selected from O, N, or S;
n is 1 to 3;
R¹ and R² are independently selected from hydrogen or a C₁₋₆ alkyl group, or together with the carbon to which they are attached join to form a C₃₋₈ cycloalkyl or a heterocyclyl group;
X is -DArg- or a bond;
-hArg- is a homoarginine residue;
AA¹ is the residue: 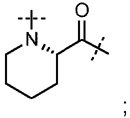
or is an aspartic acid derived residue joined to AA³ via a lactam bridge;
AA² is -Gly- or is a glutamic acid derived residue joined to AA⁵ via a lactam bridge;
AA³ is -His-, a 4-bromophenylalanine residue or is a lysine derived residue joined to AA¹ via a lactam bridge;
AA⁴ is the residue: 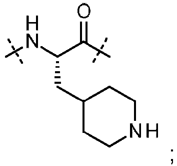
AA⁵ is -Gly- or is a lysine derived residue joined to AA² via a lactam bridge;
AA⁶ is the residue: 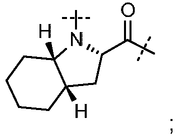
AA⁷ is a norleucine residue or a 4-bromophenylalanine residue;
AA⁸ is the residue: 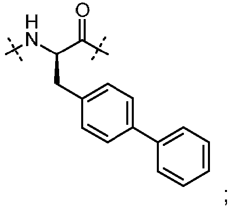
wherein the AA⁸ C-terminus is a carboxyl group or a carboxamide group and wherein the compound contains a lactam bridge;
or a tautomeric or stereochemically isomeric form thereof or a salt or zwitterion thereof.

2. The compound according to claim 1, wherein Q is:

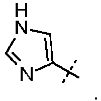

3. The compound according to claim 1 or claim 2, wherein n is 2.

4. The compound according to any one of claims 1 to 3, wherein R¹ and R² are independently selected from hydrogen or a C₁₋₆ alkyl group.

5. The compound according to claim 4, wherein R¹ and R² are both methyl.

6. The compound according to any one of claims 1 to 5, wherein X is -DArg-.

7. The compound according to any one of claims 1 to 5, wherein X is a bond.

8. The compound according to any one of claims 1 to 7, wherein AA¹ is an aspartic acid derived residue joined via a lactam bridge to AA³ which is a lysine derived residue.

9. The compound according to any one of claims 1 to 7, wherein AA² a glutamic acid derived residue joined via a lactam bridge to AA⁵ which is a lysine derived residue.

10. The compound according to any one of claims 1 to 9, wherein the AA⁸ C-terminus is a carboxyl group.

11. The compound according to claim 1 which is selected from the group consisting of:

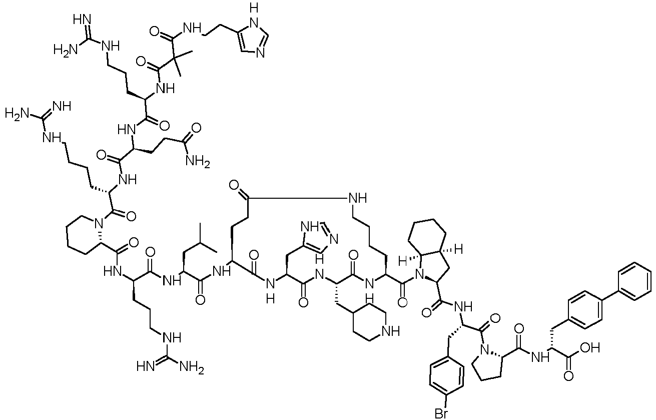

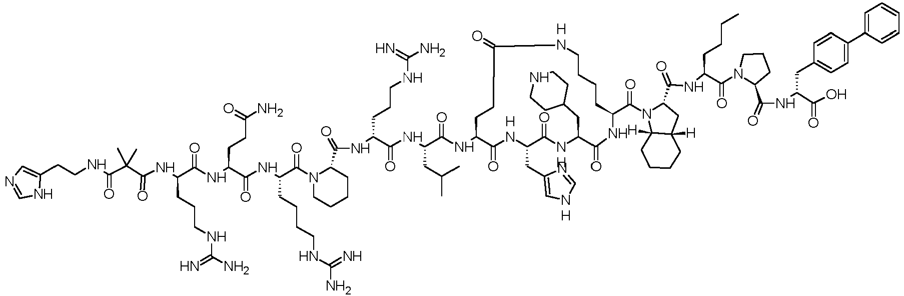

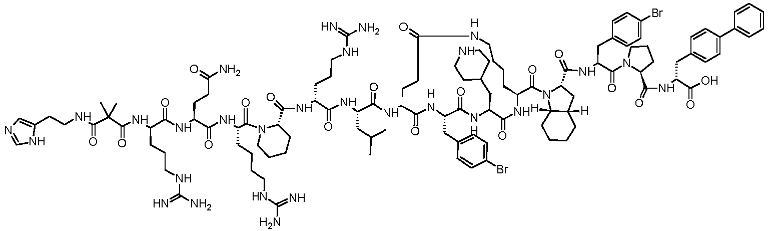

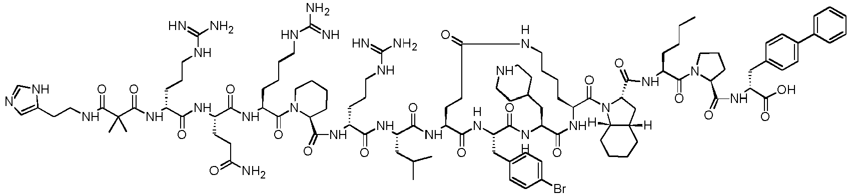

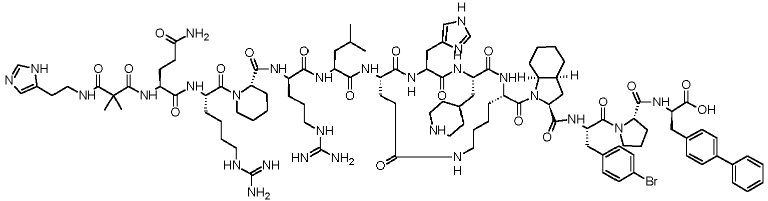

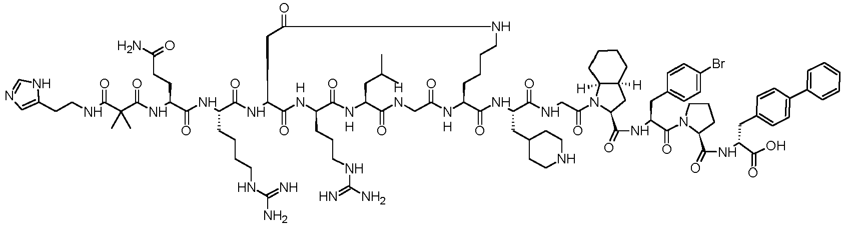

12. The compound, tautomeric or stereochemical isomeric form, salt or zwitterion according to any one of claims 1 to 11 having apelin receptor agonist activity.

13. A pharmaceutical composition comprising a compound, tautomeric or stereochemical isomeric form, salt or zwitterion as defined in any one of claims 1 to 12 and a pharmaceutically acceptable excipient.

14. The compound, tautomeric or stereochemical isomeric form, salt or zwitterion according to any one of claims 1 to 12 or composition according to claim 13 for use in medicine.

15. The compound, tautomeric or stereochemical isomeric form, salt or zwitterion according to any one of claims 1 to 12 or composition according to claim 13 for use in the treatment of cardiovascular disease, acute decompensated heart failure, congestive heart failure, myocardial infarction, cardiomyopathy, ischemia, ischemia/reperfusion injury, pulmonary hypertension, diabetes, treatment of pulmonary arterial hypertension (PAH) increasing cardiac output, reducing pulmonary vessel hypertension, improve pulmonary tissue remodelling, preserving right heart ventricular function, heart failure, congestive heart failure, cardiomyopathy, ischemia, ischemia/reperfusion injury, kidney failure, hypertension, polycystic kidney disease, hyponatremia, SIADH, peripheral arterial disease (PAD), acute coronary syndrome (ACS), heart attacks (HA), stroke, atherosclerosis, and diabetic complications such as diabetic nephropathy, retinopathy, neuropathy, non-alcoholic fatty liver disease, non-alcoholic steatosis, and portal hypertension.

## Patentansprüche

1. Verbindung, die die Sequenz der Formel (1) umfasst: 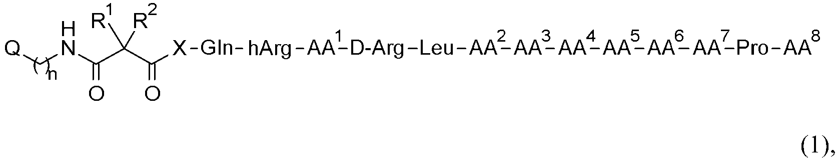 wobei:
Q aus Phenyl oder einem monocyclischen Heteroarylring ausgewählt ist, von denen jeder optional mit einer oder mehreren R⁹-Gruppen substituiert sein kann;
oder Q eine Polyetherkette der Formel -(OCH₂CH₂)ₘOCH₃ ist, wobei m 1 bis 5 ist;
R^{q} aus Folgenden ausgewählt ist: Halogen, Hydroxyl, Amino oder C₁₋₆-Alkyl, das eine Alkylkette aufweist, die optional ein oder mehrere Heteroatome enthält, die aus O, N oder S ausgewählt sind;
n 1 bis 3 ist;
R¹ und R² unabhängig aus Wasserstoff oder einer C₁₋₆-Alkylgruppe ausgewählt sind oder sich gemeinsam mit dem Kohlenstoff, an den sie gebunden sind, verbinden, um eine C₃₋₈-Cycloalkyl- oder eine Heterocyclylgruppe zu bilden;
X -DArg- oder eine Bindung ist;
-hArg- ein Homoargininrest ist;
AA¹ der folgende Rest ist: 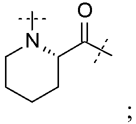
oder ein von Asparaginsäure abgeleiteter Rest ist, der über eine Lactambrücke mit AA³ verbunden ist;
AA² -Gly- ist oder ein von Glutaminsäure abgeleiteter Rest ist, der über eine Lactambrücke mit AA⁵ verbunden ist;
AA³ -His-, ein 4-Bromphenylalaninrest ist oder ein von Lysin abgeleiteter Rest ist, der über eine Lactambrücke mit AA¹ verbunden ist;
AA⁴ der folgende Rest ist: 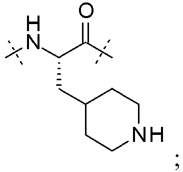
AA⁵ -Gly- ist oder ein von Lysin abgeleiteter Rest ist, der über eine Lactambrücke mit AA² verbunden ist;
AA⁶ der folgende Rest ist: 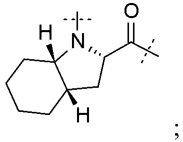
AA⁷ ein Norleucinrest oder ein 4-Bromphenylalaninrest ist;
AA⁸ der folgende Rest ist: 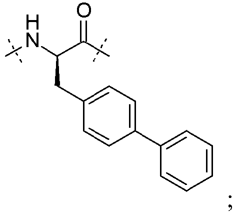
wobei das C-terminale Ende von AA⁸ eine Carboxylgruppe oder eine Carboxamidgruppe ist und wobei die Verbindung eine Lactambrücke enthält;
oder eine tautomere oder stereochemisch isomere Form davon oder ein Salz oder Zwitterion davon.

2. Verbindung nach Anspruch 1, wobei Q Folgendes ist:

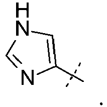

3. Verbindung nach Anspruch 1 oder Anspruch 2, wobei n 2 ist.

4. Verbindung nach einem der Ansprüche 1 bis 3, wobei R¹ und R² unabhängig aus Wasserstoff oder einer C₁₋₆-Alkylgruppe ausgewählt sind.

5. Verbindung nach Anspruch 4, wobei R¹ und R² beide Methyl sind.

6. Verbindung nach einem der Ansprüche 1 bis 5, wobei X -DArg- ist.

7. Verbindung nach einem der Ansprüche 1 bis 5, wobei X eine Bindung ist.

8. Verbindung nach einem der Ansprüche 1 bis 7, wobei AA¹ ein von Asparaginsäure abgeleiteter Rest ist, der über eine Lactambrücke mit AA³ verbunden ist, das ein von Lysin abgeleiteter Rest ist.

9. Verbindung nach einem der Ansprüche 1 bis 7, wobei AA² ein von Glutaminsäure abgeleiteter Rest ist, der über eine Lactambrücke mit AA⁵ verbunden ist, das ein von Lysin abgeleiteter Rest ist.

10. Verbindung nach einem der Ansprüche 1 bis 9, wobei das C-terminale Ende von AA⁸ eine Carboxylgruppe ist.

11. Verbindung nach Anspruch 1, die aus der Gruppe ausgewählt ist, die aus Folgenden besteht:

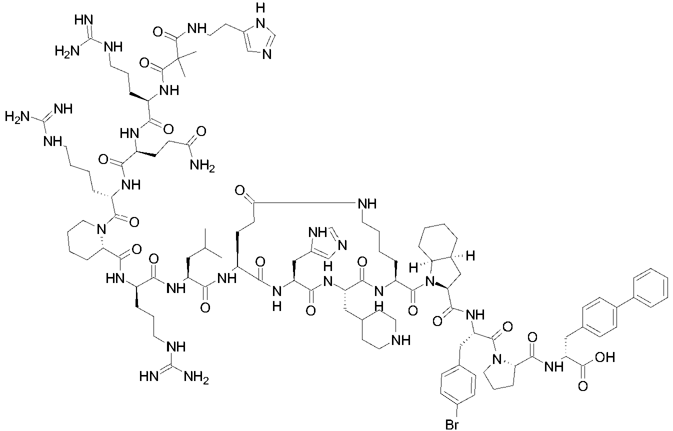

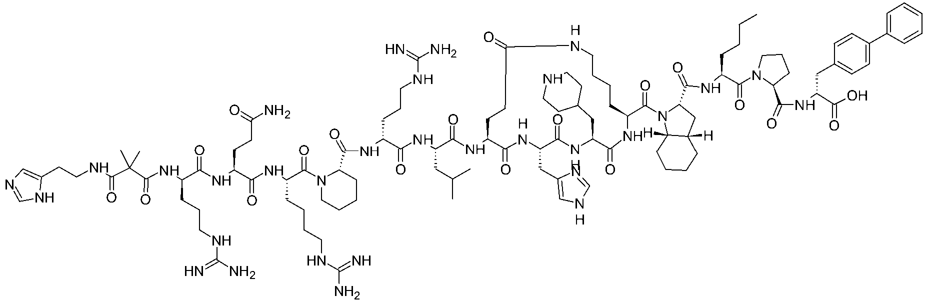

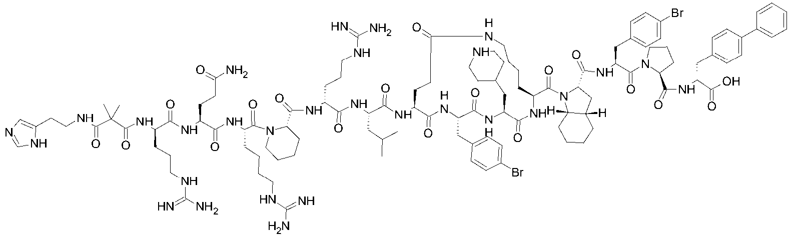

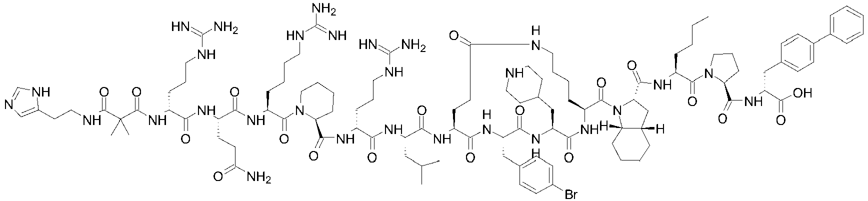

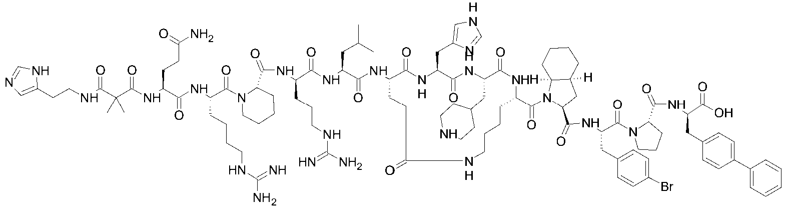

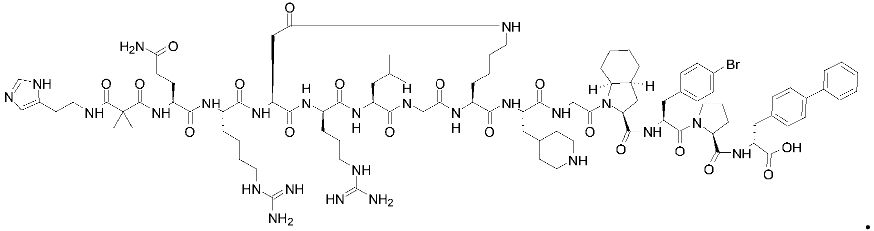

12. Verbindung, tautomere oder stereochemische isomere Form, Salt oder Zwitterion nach einem der Ansprüche 1 bis 11, die eine Apelin-Rezeptor-Agonistenaktivität aufweisen.

13. Pharmazeutische Zusammensetzung, die eine Verbindung, tautomere oder stereochemische isomere Form, ein Salt oder Zwitterion, wie in einem der Ansprüche 1 bis 12 definiert, und einen pharmazeutisch verträglichen Hilfsstoff umfasst.

14. Verbindung, tautomere oder stereochemische isomere Form, Salt oder Zwitterion nach einem der Ansprüche 1 bis 12 oder Zusammensetzung nach Anspruch 13 für die Verwendung in der Medizin.

15. Verbindung, tautomere oder stereochemische isomere Form, Salt oder Zwitterion nach einem der Ansprüche 1 bis 12 oder Zusammensetzung nach Anspruch 13 für die Verwendung bei der Behandlung von Folgendem: Herz-Kreislauf-Erkrankung, akuter dekompensierter Herzinsuffizienz, kongestiver Herzinsuffizienz, Myokardinfarkt, Kardiomyopathie, Ischämie, Ischämie-/Reperfusionsschaden, pulmonaler Hypertonie, Diabetes, Behandlung von pulmonaler arterieller Hypertonie (PAH), die die Herzleistung steigert, Reduzierung von pulmonaler Gefäßhypertonie, Verbesserung von pulmonalem Gewebeumbau, Erhaltung der Funktion des rechten Herzventrikels, Herzinsuffizienz, kongestiver Herzinsuffizienz, Kardiomyopathie, Ischämie, Ischämie-/Reperfusionsschaden, Niereninsuffizienz, Hypertonie, polyzystischer Nierenerkrankung, Hyponatriämie, SIADH, peripherer Arterienerkrankung (PAD), akutem Koronarsyndrom (ACS), Herzinfarkten (HA), Schlaganfall, Arterosklerose und diabetischen Komplikationen, wie zum Beispiel diabetischer Nephropathie, Retinopathie, Neuropathie, nichtalkoholischer Fettlebererkrankung, nichtalkoholischer Steatose und portaler Hypertonie.

## Revendications

1. Composé comprenant la séquence de la formule (1) : 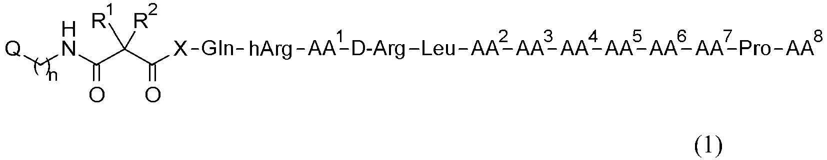 dans lequel ;
Q est sélectionné parmi phényle ou un cycle hétéroaryle monocyclique dont chacun desquels peut être optionnellement substitué par un ou plusieurs groupes R^{q} ; ou bien Q est une chaîne polyéther de la formule - (OCH₂CH₂)ₘOCH₃, où m est 1 à 5 ;
R^{q} est sélectionné parmi halogène, hydroxyle, amino ou alkyle en C₁₋₆ ayant une chaîne alkyle contenant optionnellement un ou plusieurs hétéroatomes sélectionnés parmi O, N ou S ;
n est 1 à 3 ;
R¹ et R² sont sélectionnés indépendamment parmi hydrogène ou un groupe alkyle en C₁₋₆, ou bien ils se joignent avec le carbone auquel ils sont attachés pour former un groupe cycloalkyle en C₃₋₈ ou hétérocyclyle ;
X est -DArg- ou une liaison ;
-hArg- est un résidu d'homoarginine ;
AA¹ est le résidu : 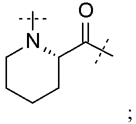
ou est un résidu dérivé d'acide aspartique joint à AA³ par un pont lactame ;
AA² est -Gly- ou est un résidu d'acide glutamique joint à AA⁵ par un pont lactame ;
AA³ est -His-, un résidu de 4-bromophénylalanine ou un résidu de lysine joint à AA¹ par un pont lactame ;
AA⁴ est le résidu : 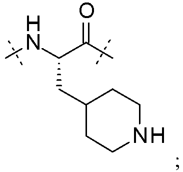
AA⁵ est -Gly- ou un résidu dérivé de lysine joint à AA² par un pont lactame ;
AA⁶ est le résidu : 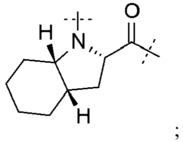
AA⁷ est un résidu de norleucine ou un résidu de 4-bromophénylalanine ;
AA⁸ est le résidu : 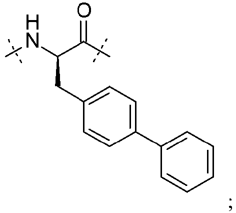
dans lequel la terminaison C de AA⁸ est un groupe carboxyle ou un groupe carboxamide et dans lequel le composé contient un pont lactame ;
ou une forme tautomère ou stéréochimiquement isomère de celui-ci ou un sel ou un zwitterion de celui-ci.

2. Composé selon la revendication 1, dans lequel Q est :

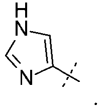

3. Composé selon la revendication 1 ou la revendication 2, dans lequel n est 2.

4. Composé selon l'une quelconque des revendications 1 à 3, dans lequel R¹ et R² sont sélectionnés indépendamment parmi hydrogène ou un groupe alkyle en C₁₋₆.

5. Composé selon la revendication 4, dans lequel R¹ et R² sont tous les deux un méthyle.

6. Composé selon l'une quelconque des revendications 1 à 5, dans lequel X est une -DArg-.

7. Composé selon l'une quelconque des revendications 1 à 5, dans lequel X est une liaison.

8. Composé selon l'une quelconque des revendications 1 à 7, dans lequel AA¹ est un dérivé d'acide aspartique joint par un pont lactame à AA³ qui est un résidu dérivé de lysine.

9. Composé selon l'une quelconque des revendications 1 à 7, dans lequel AA² est un dérivé d'acide glutamique joint par un pont lactame à AA⁵ qui est un résidu dérivé de lysine.

10. Composé selon l'une quelconque des revendications 1 à 9, dans lequel la terminaison C de AA⁸ est un groupe carboxyle.

11. Composé selon la revendication 1, qui est sélectionné parmi le groupe composé de :

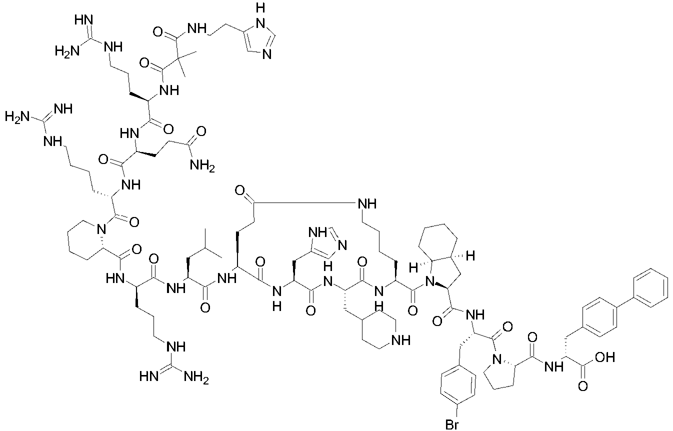

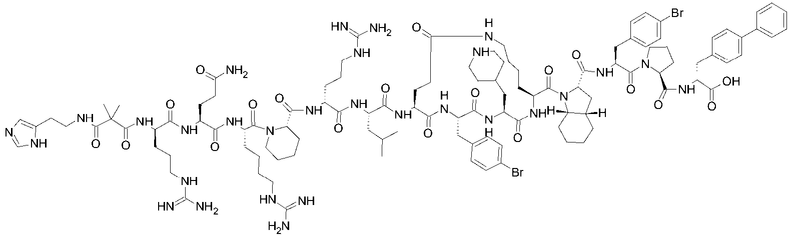

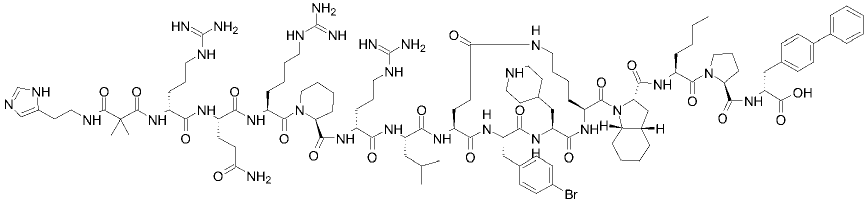

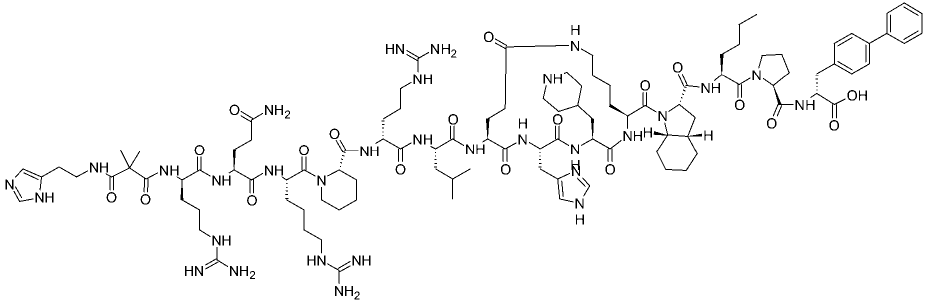

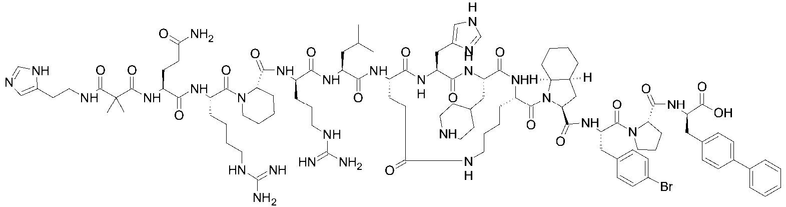

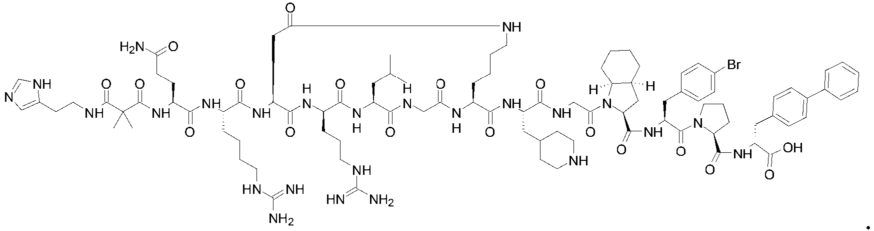

12. Composé, forme tautomère ou stéréochimique isomère, sel ou zwitterion selon l'une quelconque des revendications 1 à 11 ayant une activité d'agoniste du récepteur de l'apéline.

13. Composition pharmaceutique comprenant un composé, une forme tautomère ou stéréochimique isomère, un sel ou un zwitterion de celui-ci comme défini selon l'une quelconque des revendications 1 à 12 et un excipient pharmaceutiquement acceptable.

14. Composé, forme tautomère ou stéréochimique isomère, sel ou zwitterion selon l'une quelconque des revendications 1 à 12 ou composition selon la revendication 13 pour une utilisation en médecine.

15. Composé, forme tautomère ou stéréochimique isomère, sel ou zwitterion selon l'une quelconque des revendications 1 à 12 ou composition selon la revendication 13 pour une utilisation dans le traitement d'une maladie cardiovasculaire, d'une défaillance cardiaque décompensée aiguë, d'une défaillance cardiaque congestive, d'un infarctus du myocarde, de cardiomyopathie, ischémie, lésion d'ischémie/reperfusion, hypertension pulmonaire, diabète, traitement de l'hypertension artérielle pulmonaire (HTAP) accroissant le débit cardiaque, réduisant l'hypertension des vaisseaux pulmonaires, améliorant le remodelage tissulaire pulmonaire, préservant la fonction ventriculaire cardiaque droite, défaillance cardiaque, défaillance cardiaque congestive, cardiomyopathie, ischémie, lésion d'ischémie/reperfusion, défaillance rénale, hypertension, maladie polykystique des reins, hyponatrémie, syndrome de sécrétion inappropriée d'hormone antidiurétique, artériopathie oblitérante des membres inférieurs (PAD), syndrome coronarien aigu (ACS), crises cardiaques (CC), accident vasculaire cérébral, athérosclérose et complications diabétiques telles que néphropathie diabétique, rétinopathie, neuropathie, maladie du foie gras non alcoolique, stéatose non alcoolique et hypertension portale.
